# EUROPEAN PATENT APPLICATION

(11) **EP 3 388 517 A1**
(43) Date of publication of application: **17.10.2018**
(21) Application number: 17165784.4
(22) Date of filing: 10.04.2017
(51) Int. Cl.: C12N 9/12, A61K 31/00, A61K 31/51, C07C 259/06, C07D 401/04, C07D 487/04, C12N 15/10, C12N 15/63, C12N 15/90

(54) **COMPOUNDS FOR INCREASING GENOME EDITING EFFICIENCY**

(71) Applicant: Max-Planck-Gesellschaft zur Förderung der Wissenschaften e.V., 80539 München (DE)
(72) Inventor: RIESENBERG, Stephan, 07743 Jena (DE); MARICIC, Tomislav, 04103 Leipzig (DE)
(74) Representative: Weickmann & Weickmann PartmbB

(57) **Abstract**

Compounds, compositions and kits to increase precise genome editing efficiency in eukaryotic cells or organisms. Combinations of at least two compounds selected from: (a) HDAC inhibitor, preferably Trichostatin A, (b) NAE inhibitor, preferably MLN4924 (c) DNA-PK inhibitor, preferably NU7026 and, (d) RPA inhibitor, preferably NSC15520. Use of a catalytically inactive mutant of DNA protein kinase catalytic subunit (DNA-PKcs) K3753R, to enhance genome editing, optionally, combined with one or more of (a) to (d).

SEQUENCE LISTING

<110>  Max-Planck-Gesellschaft zur Förderung der Wissenschaften
       e.V.

<120>  Directing repair pathways with small molecules to increase
       precise genome editing efficieny

<130>  63611P EP

<160>  31

<170>  PatentIn version 3.5

<210>  1
<211>  20
<212>  DNA
<213>  Artificial Sequence

<220>
<223>  gRNA for CALD1

<400>  1
tggagactat tgctgcttga                                                   20


<210>  2
<211>  20
<212>  DNA
<213>  Artificial Sequence

<220>
<223>  gRNA for CALD1

<400>  2
gcagtatacc agtgcaattg                                                   20


<210>  3
<211>  20
<212>  DNA
<213>  Artificial Sequence

<220>
<223>  gRNA for KATNA1

<400>  3
aaatgatgac ccttccaaaa                                                   20


<210>  4
<211>  20
<212>  DNA
<213>  Artificial Sequence

<220>
<223>  gRNA for KATNA1

<400>  4
caacacctaa aataagggta                                                   20


<210>  5

<211>  20
<212>  DNA
<213>  Artificial Sequence

<220>
<223>  gRNA for SLITRK1

<400>  5
gctaacagtt taccctgccc                                                    20


<210>  6
<211>  20
<212>  DNA
<213>  Artificial Sequence

<220>
<223>  gRNA for SLITRK1

<400>  6
acccgtcgct atcgctgctg                                                    20


<210>  7
<211>  21
<212>  DNA
<213>  Artificial Sequence

<220>
<223>  gRNA for HPRT

<400>  7
ggttaaagat ggttaaatga t                                                  21


<210>  8
<211>  21
<212>  DNA
<213>  Artificial Sequence

<220>
<223>  gRNA forDMNT1

<400>  8
ctgatggtcc atgtctgtta c                                                  21


<210>  9
<211>  90
<212>  DNA
<213>  Artificial Sequence

<220>
<223>  ssODN for CALD1

<400>  9
gtatactgct ccagtctgct gtcaatcttg gagactactg ctgcttgatg ggtcgatttg        60

acaccactgc taaaaaagta aacacataca                                         90


<210>  10
<211>  153

<212>  DNA
<213>  Artificial Sequence

<220>
<223>  ssODN for CALD1

<400>  10
ttatatgtat gtgtttactt ttttagcagt ggtgtcaaat cgacccatca agctgcagta      60

gtctccaaga ttgacagcag actggagcag tataccagtg ctattgaggt gagaattgtc     120

ctcagcgtta tggtcctgct gaacagaaat aga                                  153


<210>  11
<211>  90
<212>  DNA
<213>  Artificial Sequence

<220>
<223>  ssODN for KATNA1

<400>  11
gaagggtcat cattttcaga agcacctcca acacctaaaa taagggaaag gggagagtga      60

aaaagatatt aagttggatt ataccaaatg                                       90


<210>  12
<211>  147
<212>  DNA
<213>  Artificial Sequence

<220>
<223>  ssODN for KATNA1

<400>  12
ctcatctata tcccagggaa aattagtagc tgccagaacc ataaccattt tagaagggtc      60

atcattttca gaggcgcctc caacacctaa aataacggta aggggagagt gaaaaagata     120

ttaagttgga ttataccaaa tgaagct                                         147


<210>  13
<211>  90
<212>  DNA
<213>  Artificial Sequence

<220>
<223>  ssODN for SLITRK1

<400>  13
tgttagctaa gggtttgttc ctggcgctac ccgtcgctat cgctgcggtg ggtctgattt      60

tgatctgcca gttgcctggg atctttgtac                                       90


<210>  14
<211>  155
<212>  DNA
<213>  Artificial Sequence

<220>
<223>  ssODN for SLITRK1

<400>  14
tcatctttaa acccgaccct gggatgtggt cgcagctgca gcccccagga cagggtaaac      60

tgttagctaa gggtttgttc ctggcactgc ccgtcgctat cgctgcggtg ggtctgattt     120

tgatctgcca gttgcctggg atctttgtac ctccg                                155


<210>  15
<211>  90
<212>  DNA
<213>  Artificial Sequence

<220>
<223>  ssODN for HPRT

<400>  15
gccatttcac ataaaactct tttaggttat agatggttaa atgaatgaca aaaaaagtaa      60

ttcacttaca gtctggctta tatccaacac                                       90


<210>  16
<211>  90
<212>  DNA
<213>  Artificial Sequence

<220>
<223>  ssODN for DMNT1

<400>  16
ttaacatcag tacgttaatg tttcctgatc gtccatgtct gttagtcgcc tgtcaagtgg      60

cgtgacaccg ggcgtgttcc ccagagtgac                                       90


<210>  17
<211>  60
<212>  DNA
<213>  Artificial Sequence

<220>
<223>  forward primer for CALD1

<400>  17
acactctttc cctacacgac gctcttccga tctgctaatc agctagcata tgtatgagaa      60


<210>  18
<211>  59
<212>  DNA
<213>  Artificial Sequence

<220>
<223>  reverse primer for CALD1

<400>  18
gtgactggag ttcagacgtg tgctcttccg atctttggac ttgattattg tcctaagtg       59

<210>  19
<211>  54
<212>  DNA
<213>  Artificial Sequence

<220>
<223>  forward primer for KATNA1

<400>  19
acactctttc cctacacgac gctcttccga tctcctgacg gcaaaggaat atag          54


<210>  20
<211>  57
<212>  DNA
<213>  Artificial Sequence

<220>
<223>  reverse primer for KATNA1

<400>  20
gtgactggag ttcagacgtg tgctcttccg atctactgtg cttccttgta ttgttgt       57


<210>  21
<211>  52
<212>  DNA
<213>  Artificial Sequence

<220>
<223>  forward primer for SLITRK1

<400>  21
acactctttc cctacacgac gctcttccga tctgggcttc aaatcagcca ag            52


<210>  22
<211>  54
<212>  DNA
<213>  Artificial Sequence

<220>
<223>  reverse primer for SLITRK1

<400>  22
gtgactggag ttcagacgtg tgctcttccg atcttttcaa gacaaatggg caag          54


<210>  23
<211>  53
<212>  DNA
<213>  Artificial Sequence

<220>
<223>  forward primer for HPRT

<400>  23
acactctttc cctacacgac gctcttccga tctggtgaaa aggaccccac gaa           53


<210>  24
<211>  58
<212>  DNA

<213>  Artificial Sequence

<220>
<223>  reverse primer for HPRT

<400>  24
gtgactggag ttcagacgtg tgctcttccg atcttggcaa atgtgcctct ctacaaat          58


<210>  25
<211>  55
<212>  DNA
<213>  Artificial Sequence

<220>
<223>  forward primer for DMNT1

<400>  25
acactctttc cctacacgac gctcttccga tcttgaacgt tcccttagca ctctg             55


<210>  26
<211>  54
<212>  DNA
<213>  Artificial Sequence

<220>
<223>  reverse primer for DMNT1

<400>  26
gtgactggag ttcagacgtg tgctcttccg atctccttag cagcttcctc ctcc              54


<210>  27
<211>  23
<212>  DNA
<213>  Artificial Sequence

<220>
<223>  forward primer for D10A

<400>  27
tggtgccgat agccaggccg atg                                                23


<210>  28
<211>  18
<212>  DNA
<213>  Artificial Sequence

<220>
<223>  reverse primer for D10A

<400>  28
actctgtggg ctgggccg                                                      18


<210>  29
<211>  69
<212>  DNA
<213>  Homo sapiens

<400>  29

acccatcaag cagcaatagt ctccaagatt gacagcagac tggagcagta taccagtgca 60

attgaggtg 69

<210> 30
<211> 63
<212> DNA
<213> Homo sapiens

<400> 30
aaccattttg gaagggtcat cattttcaga agtacctcca acacctaaaa taagggtaag 60

ggg 63

<210> 31
<211> 71
<212> DNA
<213> Homo sapiens

<400> 31
ccccagggca gggtaaactg ttagctaagg gtttgttcct ggagctaccc gtcgctatcg 60

ctgctgtggg t 71

## Description

The present invention relates to compounds, compositions and kits suitable to increase precise genome editing efficiency in a eukaryotic target cell or target organism.

CRISPR is a bacterial nuclease of the immune system against viral DNA, which has been adopted to accurately cut chromosomal DNA sequences in eukaryotic cells. Such DNA breaks are repaired by two competing pathways: Non-homologous-End-Joining (NHEJ) or Homology directed Repair (HDR).

In NHEJ, the first proteins to bind to the DNA ends are Ku70/Ku80, followed by DNA protein kinase catalytic subunit (DNA-PKcs) (Shrivastav et al. 2008). The kinase phosphorylates itself and other downstream effectors at the repair site. Recruitment and phosphorylation of several proteins like Artemis result in end-processing ligation by ligase IV (LIG4), X-ray repair cross-complementing protein 4 (XRCC4) and Non-homologous end-joining factor 1 (XLF) (Dueva, Iliakis 2013).

If this canonical NHEJ pathway is repressed, an alternative NHEJ pathway (A-NHEJ) becomes active (Nussenzweig, Nussenzweig 2007). It requires Poly(ADP-ribose)-Polymerase 1 (PARP-1), Werner syndrome ATP-dependent (WRN) helicase and DNA ligase 3 (LIG3) or DNA ligase I (LIG1) amongst other proteins. Binding of the MRN-complex (Mre11, Rad50 and Nbs1) complex to the double strand break (DSB) initiates HDR (Shrivastav et al. 2008). Along with other proteins like DNA endonuclease RBBP8 (CtIP), Bloom helicase (BLM) and Exonuclease 1 (EXO1), terminal nucleotides in the 5' ends are removed, generating long 3' single-stranded DNA (ssDNA) overhangs on both sides of the break of the DNA (Dueva, Iliakis 2013). These tails are then coated and stabilized by the Replication protein A (RPA) complex, followed by breast cancer 2 (BRCA2) assisted generation of a Rad51 nucleoprotein filament (Shrivastav et al. 2008). Rad52 facilitates replacement of RPA bound to ssDNA with Rad51 and promotes ssDNA annealing (Grimme et al. 2010). Strand invasion with the donor DNA and subsequent DNA synthesis by a polymerase finally results in precisely repaired DNA. The protein kinase ataxia-telangiectasia mutated (ATM) plays a major role in HDR, as it phosphorylates at least 12 repair proteins (Shrivastav et al. 2008).

NHEJ of CRISPR Cas9-induced DSBs is error prone and frequently introduces insertions and deletions (indels) at the cut site. It is therefore useful for knocking out a targeted gene. In contrast, HDR allows precise repair of a DSB by using a homologous donor DNA sequence. If this donor sequence is provided in the experiment and carries mutations, these will be introduced into the genome.

A requirement for a DSB introduced by Cas9 is an NGG sequence (PAM site) in DNA. Targeting of Cas9 is determined by a bound guide RNA (gRNA) which is complementary to 20 nucleotides adjacent to the PAM site. However, the Cas9 nuclease may also cut the genome at sites that carry sequence similarity to those targeted by the gRNA (Fu et al. 2013).Those off-target double stranded cuts mean that unwanted mutations can appear elsewhere in the genome together with the desired mutation.

One strategy to reduce such off-target cuts is to use a mutated Cas9 that introduces single-stranded nicks instead of DSBs such as Cas9 D10A (Shen et al. 2014). Using two gRNAs to introduce two nicks on opposite DNA strands in close proximity to each other will result in a DSB at the desired locus while reducing the risk of two off-target nicks occurring elsewhere in the genome close enough to cause a DSB. Another strategy is to use Cpf1 (Zetsche et al. 2015). This nuclease introduces a staggered cut near a T-rich PAM site and has been shown to produce less off-target effects (Kim et al. 2016)(Kleinstiver et al. 2016).

In current approaches, precise genome editing (PGE) efficiencies, especially for targeted nucleotide substitutions in stem cells, are usually low, ranging from 0.5-15% (Yu et al. 2015)(Gonzalez et al. 2014). Several researchers addressed the low rate of precise genome editing by trying to promote HDR or decrease NHEJ.

Cell cycle synchronization to G2/M phase was shown to increase PGE with single stranded oligodeoxynucleotide (ssODN) donors in HEK293T cells (from 26% to 38%), human primary neonatal fibroblasts (from undetectable to 0.6%) and human embryonic stem cells (hESCs) (from undetectable to 1.6%) (Lin et al. 2014) and with double stranded oligodeoxynucleotide (dsODN) donors in hESCs (from 7 to 41%)(Yang et al. 2016), since homologous recombination is restricted to this phase and its proteins are upregulated.

Also, improved efficiency was achieved by suppressing key proteins like Ku70/80 and ligase IV with siRNA (from 5 to 25%) or co-expression of adenovirus type 5 proteins 4E1B55K and E4orf6 (from 5 to 36%) in HEK293/TLR cells using dsODN donors (Chu et al. 2015). E1 B55K and E4orf6 proteins mediate the ubiquitination and proteosomal degradation of LIG4 among other targets.

A common strategy to increase genome editing has been the use of small molecules. The small molecule ligase IV inhibitor SCR7 has been claimed to block NHEJ and to increase the efficiency of PGE (from 5 to 22.7%) in mouse embryos (Maruyama et al. 2015). Other researchers described similar increase in HEK293/TLR cells, a marginal but significant increase in HEK293A, or found no significant effect in mouse embryos, rabbit embryos and human stem cells (Chu et al. 2015)(Pinder et al. 2015)(Song et al. 2016)(Yang et al. 2016)(Zhang et al. 2017). Recently, Greco et al. reanalysed the structure and inhibitory properties of SCR7 (Greco et al. 2016). They conclude that SCR7 and its derivates are neither selective nor potent inhibitors of human LIG4.

Pharmacological inhibition of DNA-PK, a key protein complex in the NHEJ-pathway, by the small molecules NU7441, KU-0060648 and NU7026 was shown to reduce the frequency of NHEJ and to increase PGE in HEK293/TLR cells (from 1.9 to 3.8%), HEK293 (3 to 7.6%) and human induced pluripotent stem cells (hiPSCs) (from 13 to 16%) with dsODN donors and in mouse embryonic fibroblasts (from 3 to 10%) with ssODN donors (Robert et al. 2015)(Suzuki et al. 2016)(Zhang et al. 2017).

Also, a single small molecule enhancing homologous recombination with CRISPR-Cas9 has been described. The RAD51 stimulatory compound RS-1 increased PGE in rabbit embryos (from 4.4 to 26.1 %), HEK293A cells (from 3.5 to 21 %) and U2OS cells (from 1.9 to 2.4%)(Song et al. 2016)(Pinder et al. 2015), but not in hiPSCs (Zhang et al., 2017), all with dsODN donors. No effect of RS-1 on PGE efficiency was found in porcine fetal fibroblasts using ssODN donors (Wang et al. 2016).

Furthermore, using a library screen of around 4000 small molecules, Yu et al. found the β3-adrenergic receptor agonist L755507 to increase PGE in hiPSCs (from 0.35 to 3.13%) using ssODN and using dsODN donors in mouse ESCs (from 17.7 to 33.3%), while the repair pathway target of that molecule is not known (Yu et al. 2015). Others did not find significant stimulation of PGE by L755507 in HEK293A cells or hiPSCs (Pinder et al. 2015)(Zhang et al. 2017). Pinder et al. compared SCR7, RS-1 and L755507 singly and together and found no additive effect when adding SCR7 and L755507 together with RS-1 compared to RS-1 alone.

From the overview of the current state-of-the art of small molecules that enhance CRIPR-Cas9 genome editing we learn that inhibitors of DNA-PK may increase PGE in CRISPR-Cas9 genome editing, but that the effects of SCR7, L755507 and RS-1 were not consistent between cell lines and loci. We also learn that previously tested combinations of small molecules did not show an additive effect.

The present inventors have found that certain compounds, particularly when applied as a combination of two or more different compounds increase precise genome editing efficiency. In particular, the inventors have found that compounds selected from inhibitors of histone deacetylase (HDAC) inhibitors of NEDD8 activating enzyme (NAE), inhibitors of DNA-dependent Protein Kinase (DNA-PK) in particular of its catalytic subunit (DNA-PKcs), and inhibitors of replication protein A (RPA) and combinations of compounds selected from these different classes of inhibitors, are capable of increasing genome editing efficiency. The compounds and combinations thereof are suitable both in non-medical applications, e.g. as research tool or in medical applications, e.g. for in vivo or ex vivo use.

In a first aspect, the invention relates to a compound which is an inhibitor of histone deacetylase (HDAC), in the following designated as compound (I), for use in genome editing.

HDAC inhibitors are known as cytostatic agents for inhibiting tumor cell proliferation by inducing cell cycle arrest, differentiation and/or apoptosis. HDAC inhibitors usually act by binding to the zinc-containing catalytic domain of HDACs. They may be classified according to the chemical moiety that binds to the zinc ion. Examples of suitable classes of HDAC inhibitors are:
(1) Hydroxamate compounds,
(2) Cyclic tetrapeptides and depsipeptides which bind to the zinc ion via a thiol group,
(3) Benzamide compounds,
(4) Electrophilic ketones and
(5) Aliphatic acid compounds.

HDAC inhibitors are reviewed e.g. by Khan & La Thangue (Immunol. Cell Biol. 90 (2012), 85-94) and Falkenberg & Johnstone (Nature Rev. Drug Discovery 13 (2014) 673-691), herein incorporated by reference.

According to the present invention, HDAC inhibitors are preferably selected from synthetic non-nucleosidic compounds, e.g. small molecules having a molecular mass of 1500 Da or less or 1000 Da or less. Specific examples of HDAC inhibitors are selected from Trichostatin A, Vorinostat, Entinostat, Panobinostat, Mocetinostat, Belinostat, Romidepsin, MC1568, Tubastatin A HCl, Givinostat, LAQ824, CUDC-101, Quisinostat 2HCl, Pracinostat, PCI-34051, Droxinostat, PCI-24781, RGFP966, AR-42, Rocilinostat, Valproic acid, Cl994, CUDC-907, Tubacin, M344, Resminostat, RG2833, Divalproex Sodium, Scriptaid, Phenylbutyrate, Tubastatin A, CAY10603, Nexturastat A, BG45, LMK-235, Santacruzamate A, BRD73954, HPOB, TMP269, Tasquinimod and 4SC-202 as well as salts or solvates thereof, in particular pharmaceutically acceptable salts or solvates thereof.

A preferred compound (I) is Trichostatin A including salts and solvates thereof.

In a second aspect, the present invention relates to a compound which is an inhibitor of NEDD8 activating enzyme (NAE), in the following designated as compound (II), for use in genome editing.

NAE inhibitors are known as anti-tumor agents as reviewed e.g. by Nawrocki et al. (Exp Opin Investing Drugs 21(2012), 1564-1573) or as antiviral agents as reviewed e.g. by Le-Trilling et al. (Sci. Rep. 6 (2016), doi: 19977), herein incorporated by reference.

According to the present invention, NAE inhibitors are preferably selected from synthetic non-nucleosidic compounds, e.g. small molecules having a molecular mass of 1500 Da or less or 1000 Da or less. A preferred NAE inhibitor is MLN4924 (Pevonedistat) or any salt or solvate thereof, in particular any pharmaceutically acceptable salt or solvate thereof.

In a third aspect, the present invention relates to a compound which is an inhibitor of DNA-dependent protein kinase (DNA-PK), in particular an inhibitor of its catalytic subunit (DNA-PKcs), in the following designated as compound (III) for use in genome editing.

DNA-PK inhibitors are known as chemotherapeutic agents as reviewed e.g. by Davidson et al. (Front. Pharmacol. 4(2013), doi: 13 3389), herein incorporated by reference.

According to the present invention, DNA-PK inhibitors are preferably selected from synthetic non-nucleosidic compounds, e.g. small molecules having a molecular mass of 1500 Da or less or 1000 Da or less. Specific examples of DNA-PK inhibitors are NU7026, NU7441, PIK-75 and PI-103 as well as salts or solvates thereof, in particular pharmaceutically acceptable salts and solvates thereof.

In a preferred embodiment, the compound (III) is NU7026 including salts and solvates thereof.

In a fourth aspect, the invention relates to a compound which is an inhibitor of Replication Protein A (RPA), in the following designated as compound (IV) for genome editing.

RPA inhibitors are known as anti-tumor agents as reviewed e.g. by Neher et al. (Mel. Cancer Ther. 10(2011), 1756-1806), herein incorporated by reference.

According to the present invention, RPA inhibitors are preferably selected from synthetic non-nucleosidic compounds, e.g. small molecules having a molecular mass of 1500 Da or less or 1000 Da or less. Specific examples of RPA inhibitors are NSC15520, TDRL-505 and NSC111847, as well as salts or solvates thereof, in particular pharmaceutically acceptable salts and solvates thereof.

A preferred embodiment of compound (IV) is NSC15520 including salts and solvates thereof.

The present inventors have found that a compound (I), a compound (II), a compound (III), or a compound (IV) increase the frequency of precise genome editing in a eukaryotic cell such as an animal, e.g. mammalian including human cell.

In particular, the inventors found that compounds (I), (II), (III), and/or (IV) have an additive effect when administered together. In particular, when using a combination consisting of compounds Trichostatin A, MLN4924, NSC15520, and NU7026, an increase in precise genome editing of up to 6.7 fold or almost 50% edited chromosomes was achieved, the highest genome editing efficiency of human pluripotent stem cells described to date to the inventors' knowledge. Furthermore, when using the above combination of compounds in a pluripotent stem cell with a catalytically inactive DNA protein kinase catalytic subunit, in particular the mutant K3753R, an almost complete correct genome editing with up to 82% edited chromosomes corresponding to a 19.2 fold increase is achieved.

An especially strong additive effect of administering a combination of two or more of compounds (I), (II), (III), and (IV) was found together with the use of a nuclease (e.g. Cpf1) or nickase enzyme system (e.g. Cas9D10A) capable of introducing a staggered cut in a DNA double strand, e.g. a chromosomal DNA, at a desired locus.

Thus, the invention relates to a combination, e.g. a composition or a kit comprising at least two of (a) a compound (I), (b) a compound (II), (c) a compound (III), and (d) a compound (IV). A preferred embodiment is a combination wherein the compound (I) is Trichostatin A, and/or the compound (II) is MLN4924, and/or the compound (III) is NU7026, and/or the compound (IV) is NSC15520. In particular, the combination of the invention is intended for use in genome editing both in non-medical applications and in medical applications.

The term "combination" in the context of the present invention encompasses compositions comprising at least two compounds as indicated above together in admixture optionally together with a suitable carrier, e.g. a pharmaceutically acceptable carrier. The term "combination" also encompasses kits comprising at least two compounds as indicated above in separate forms, each optionally together with a suitable carrier, e.g. a pharmaceutically acceptable carrier.

Further, the present invention relates to a combination, e.g. a composition or kit comprising (i) at least one compound (I) and at least one compound (II), (ii) at least one compound (I) and at least one compound (III), (iii) at least one compound (I) and at least one compound (IV), (iv) at least one compound (II) and at least one compound (III), (v) at least one compound (II) and at least one compound (IV), or (vi) at least one compound (III) and at least one compound (IV). Preferred compounds (I), (II), (III), and/or (IV) are as indicated above.

Furthermore, the present invention relates to a combination, e.g. a composition or kit comprising (i) at least one compound (I), at least one compound (II), and at least one compound (III), (ii) at least one compound (I), at least one compound (II), and at least one compound (IV), or (iii) at least one compound (II), at least one compound (III), and at least one compound (IV). Preferred compounds (I), (II), (III), and/or (IV) are as indicated above.

Furthermore, the present invention relates to a combination, e.g. a composition or kit comprising at least one compound (I), at least one compound (II), at least one compound (III), and at least one compound (IV). Preferred compounds (I), (II), (III), and/or (IV) are as indicated above.

The combination of the present invention as described may further include one or more additional compounds. In one embodiment, the combination may include a compound for synchronizing cells in the G2/M phase such as Nocodazole and ABT-751 (Yang et al., 2016), paclitaxel (Shu et al., Apoptosis 2 (1997), 463-470), or colchicine or vincristine (Blajeski et al., J. Clin. Invest. 110 (2002), 91-95), or salts or solvates thereof. In a further embodiment, the combination may include an Alt-NHEJ inhibitor such as NSC19630 or a salt or solvate thereof, in particular together with a catalytically inactive DNA protein kinase catalytic subunit.

The combination e.g. the composition or kit of the invention is suitable for use in genome editing in a eukaryotic target cell, particularly in an animal target cell such as a mammalian target cell, e.g. a human target cell but also in a plant target cell including a human stem cell, for example an embryonic stem cell or a pluripotent stem cell,. Particularly, the target cell is a stem cell of a eukaryotic target organism, including an induced or embryonic pluripotent stem cell such as a human induced or embryonic pluripotent stem cell.

The combination e.g. the composition or kit of the invention is particularly suitable in a genome editing procedure which comprises introducing a staggered cut, particularly a staggered cut with 5' overhangs, into the genome of the target cell. In order to achieve this result, the target cell may comprise a mutated nickase version of CRISPR/Cas9 such as CRISPR/Cas9 D10A or CRISPR/Cas9 H840A enzymes which are mutated nickase versions of CRISPR/Cas9 or a CRISPR/Cpf1 enzyme. Alternatively, other genome editing enzymes, e.g. CRISPRs, transcription activator-like effector-based nucleases (TALENs), zinc finger nuclease proteins, Argonaute of the bacterium Thermus thermophiles (TtAgo), recombinases, or meganucleases or other enzymes may be present, particularly enzymes which provide staggered cuts in a double stranded target DNA. The present invention is also suitable together with split-fusion versions of the above enzymes, e.g. split-fusion versions of Cas9 or Cas9 D10A (Zetsche et al., 2015). The enzyme(s) may be introduced into the target cell as such, e.g. as protein or ribonucleoprotein or as nucleic acid molecule encoding the respective enzyme(s). The nucleic acid molecule may be introduced as an expression vector such as a plasmid in operative linkage with appropriate expression control elements for transient or stable expression in the target cell. Suitable transfection techniques for introducing proteins or nucleic acids into the eukaryotic target cells are well known in the art and include lipofection, electroporation, e.g. nucleofection, Ca-phosphate or virus-based methods.

The combination, e.g. composition or kit of the present invention may further comprise (i) a DNA protein kinase (DNA-PK) catalytic subunit which is catalytically inactive, but structurally intact, (ii) a nucleic acid molecule encoding the DNA protein kinase catalytic subunit of (i) and/or (iii) a eukaryotic cell capable of expressing the DNA protein kinase catalytic subunit of (i) including a split-fusion version thereof. Preferably, the catalytically inactive, but structurally intact mutant has an amino acid sequence identity to the corresponding wild-type sequence, e.g. the human sequence NP_008835.5 of at least 80%, at least 85%, at least 90%, or at least 95%. The catalytically inactive, but structurally intact DNA-PKcs subunit may be introduced into the target cell as protein or as nucleic acid encoding the respective subunit for transient or stable expression in the target cell.

In particular, the DNA-PK mutant comprises at least one mutation in the catalytic loop (amino acids 3919-3927) including the catalytic triade (N3927, D3922, H3924) or in the P-loop (amino acids 3729-3735) or in the adjacent region (amino acids 3736-3760) including amino acids F3946, T3950, and particularly K3753.

Even more particularly, the DNA-PK mutant comprises at least one mutation at position K3753, e.g. the mutation K3753R, and/or K3753H at position T3950, e.g. the mutation T3950D, and/or at position F3946, e.g. F3946D, and/or at position S1003, e.g. S1003D based on the NCBI reference sequence NP_008835.5.

The combination, e.g. the composition or kit is suitable for use with all kinds of donor nucleic acid molecules including but not limited to single stranded molecules or double stranded DNA molecules whether amplified in vivo or in vitro or chemically synthesized. The length of the donor nucleic acid molecules is usually in the range of about 20 to 200 nt, e.g. about 80 to 120 nt. The donor nucleic acid molecules are designed to include at least one desired mutation in view of the wild type sequence which is to be introduced into the genome of the target cell by genome editing.

A further aspect of the present invention relates to the use of (i) a DNA protein kinase catalytic subunit which is catalytically inactive, but structurally intact, particularly the mutated subunit K3753R, (ii) a nucleic acid molecule encoding the DNA protein kinase catalytic subunit of (i) and/or (iii) a eukaryotic cell capable of expressing the DNA protein kinase catalytic subunit of (i) for genome editing in a eukaryotic target cell, particularly in a mammalian target cell, e.g. a human target cell including a human stem cell.

Still a further aspect of the present invention is the use of the combination, e.g. the composition or kit comprising at least two different compounds (I), (II), (III), and (IV) in medicine including human or veterinary medicine. An effective dose of the compounds according to the invention, or their salts, solvates or prodrugs thereof is used, in addition to physiologically acceptable carriers, diluents and/or adjuvants for producing a pharmaceutical composition. The dose of the active compounds can vary depending on the route of administration, the age and weight of the patient, the nature and severity of the diseases to be treated, and similar factors. The daily dose can be given as a single dose, which is to be administered once, or be subdivided into two or more daily doses, and is as a rule 0.001-2000 mg. Particular preference is given to administering daily doses of 0.1-500 mg, e.g. 0.1-100 mg.

Suitable administration forms are oral, parenteral, intravenous, transdermal, topical, inhalative, intranasal and sublingual preparations. Particular preference is given to using oral, parenteral, e.g. intravenous or intramuscular, intranasal preparations, e.g. dry powder or sublingual, of the compounds according to the invention. The customary galenic preparation forms, such as tablets, sugar-coated tablets, capsules, dispersible powders, granulates, aqueous solutions, alcohol-containing aqueous solutions, aqueous or oily suspensions, syrups, juices or drops, can be used.

Solid medicinal forms can comprise inert components and carrier substances, such as calcium carbonate, calcium phosphate, sodium phosphate, lactose, starch, mannitol, alginates, gelatine, guar gum, magnesium stearate, aluminium stearate, methyl cellulose, talc, highly dispersed silicic acids, silicone oil, higher molecular weight fatty acids, (such as stearic acid), gelatine, agar agar or vegetable or animal fats and oils, or solid high molecular weight polymers (such as polyethylene glycol); preparations which are suitable for oral administration can comprise additional flavourings and/or sweetening agents, if desired.

Liquid medicinal forms can be sterilized and/or, where appropriate, comprise auxiliary substances, such as preservatives, stabilizers, wetting agents, penetrating agents, emulsifiers, spreading agents, solubilizers, salts, sugars or sugar alcohols for regulating the osmotic pressure or for buffering, and/or viscosity regulators.

Preparations for parenteral administration can be present in separate dose unit forms, such as ampoules or vials. Use is preferably made of solutions of the active compound, preferably aqueous solution and, in particular, isotonic solutions and also suspensions. These injection forms can be made available as ready-to-use preparations or only be prepared directly before use, by mixing the active compound, for example the lyophilisate, where appropriate containing other solid carrier substances, with the desired solvent or suspending agent.

Intranasal preparations can be present as aqueous or oily solutions or as aqueous or oily suspensions. They can also be present as lyophilisates which are prepared before use using the suitable solvent or suspending agent.

Inhalable preparations can present as powders, solutions or suspensions. Preferably, inhalable preparations are in the form of powders, e.g. as a mixture of the active ingredient with a suitable formulation aid such as lactose.

The preparations are produced, aliquoted and sealed under the customary antimicrobial and aseptic conditions.

The compounds of the invention may be administered alone or as a combination therapy with further active agents.

The medical use of the combination of the present invention particularly encompasses target gene therapy, e.g. the treatment of disorders associated with an undesired genotype of a patient in need of the treatment. For example, the disorder is a metabolic dysfunction or cancer. By means of the invention, cells from the patient may be subjected to a genome editing procedure in the presence of a combination as described above, thereby increasing the precise genome editing efficiency. This procedure may be carried out in vivo, i.e. by administering the combination to the patient or ex vivo with cells isolated from the patients, which are - after successful genome editing - reimplanted into the patient. The patient may be a vertebrate animal such as a mammal, preferably a human patient. Finally, the combination of the present invention is also suitable for genome editing in plant cells or plants.

Further, the invention shall be explained in more detail by the following Figures and Examples.

### Figure Legends

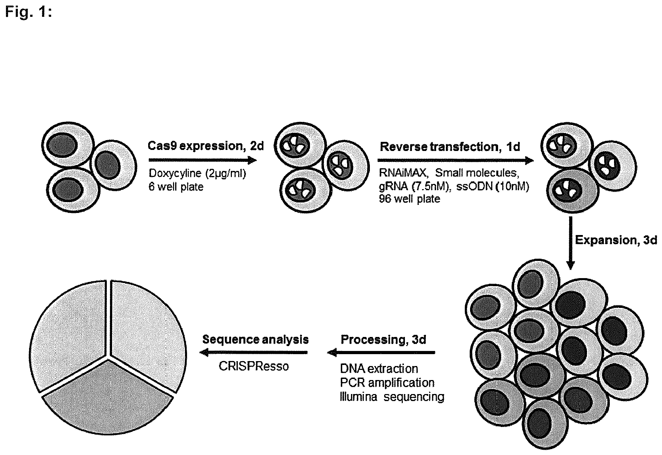

**Fig. 1****: Genome editing and analysis flowchart.** iCRISPR 409-B2 iPSCs are treated with 2 µg/ml doxycycline for 2 days to induce Cas9 or Cas9D10A expression. Reverse transfection with RNAiMAX, gRNA (7.5 nM each), ssODN (10 nM) and the small molecules to be evaluated is carried out in a 96 well plate for 1 day. The amount of cells used gives ∼ 80% confluency. Cells are then expanded for 3 days with regular media change. After harvest follows DNA extraction, PCR amplification of targeted loci, Illumina sequencing and CRISPResso (Pinello et al. 2016) sequence analysis for amount of indels and precise genome editing.

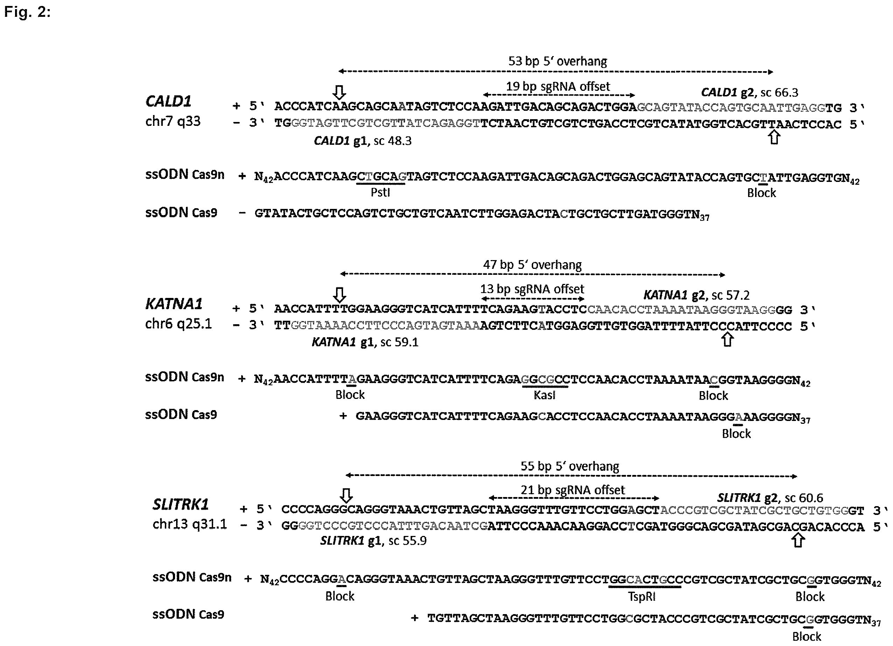

**Fig. 2****: Design of gRNAs and ssODN for precise genome editing (PGE) of human *CALD1*, *KATNA1* and *SLITRK1* to the ancient state of the last common ancestor of human and Neanderthal.** Shown are the respective loci of *CALD1*, *KATNA1* and *SLITRK1* together with the gRNAs and their efficiency score (sc) (sgRNA scorer 1.0 Chari et al. 2015) used for DSB generation. The PAM site is grey, the target sequence is blue and the base to be changed is red. The point of nick by Cas9D10A (Cas9n) or DSB by Cas9 is indicated by an arrow. Whereas both guides are used for editing with Cas9n, *CALD1* g1, *KATNA1* g2 and *SLITRK1* g2 are used for editing with Cas9. The respective ssODN for editing with both Cas9 variants are also shown. Desired mutation is marked green and additional mutations are orange. ,Block' indicates a Cas9-blocking mutation to prevent re-cutting oft the locus. All Cas9D10A donors have 50nt homology arms after the nicks while all Cas9 donors are 90nt in total with the desired mutation centered in the middle. The full sequences are shown in Table 2.

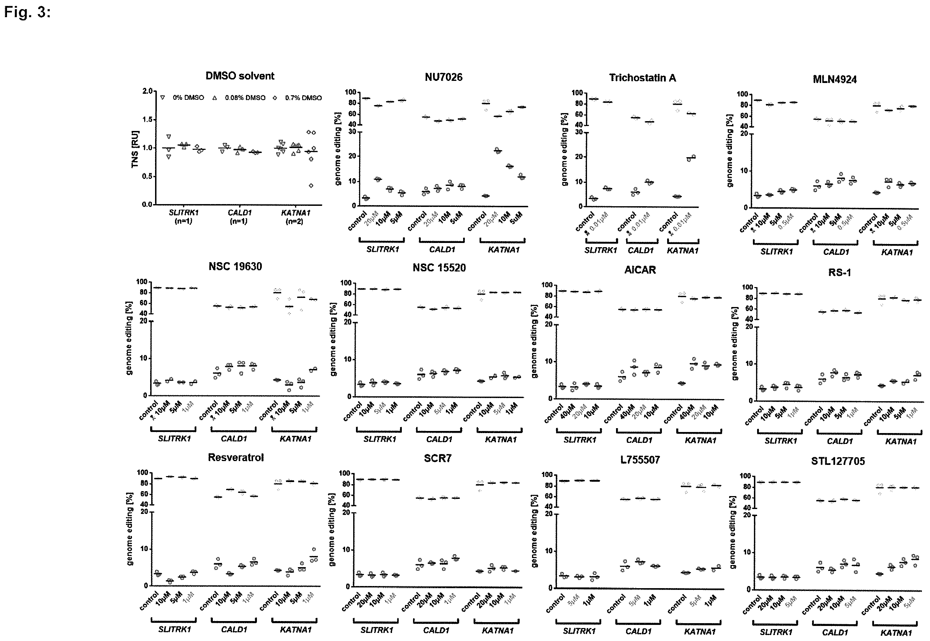

**Fig. 3****: First screen for solvent effect and influence of different small molecule concentrations on genome editing of *CALD1*, *KATNA1* and *SLITRK1* with iCRISPR Cas9D10A**. Shown are precise genome editing (PGE) and indels with green circles (triangles) or blue diamonds, respectively. Each symbol represents a technical replicate. The respective means are shown as a black line. Each skull indicates cell death of at least 20% determined by light microscopy. Concentrations chosen for further experiments are marked with turquoise.

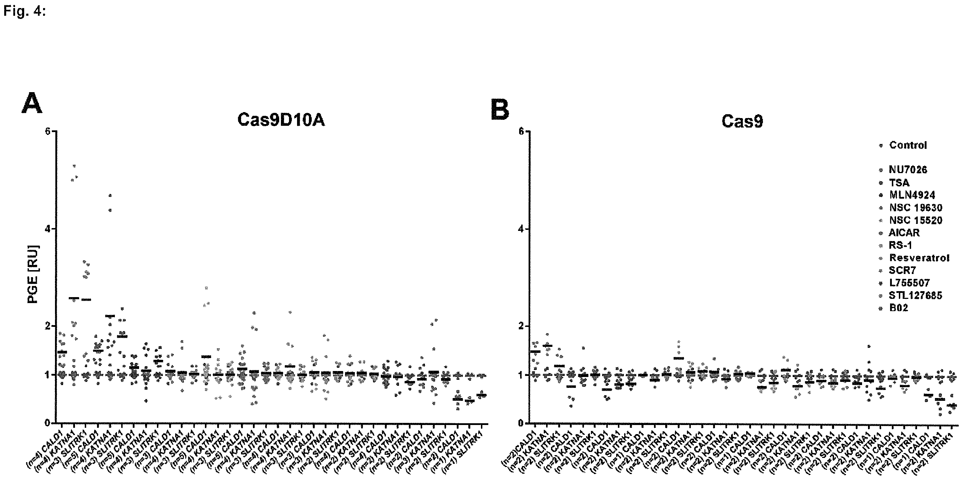

**Fig. 4****: Effects of small molecules on precise genome editing (PGE) efficiency in *CALD1*, *KATNA1* and *SLITRK1* with Cas9D10A and Cas9.** PGE efficiency is given in relative units (RU) with the mean of controls set to 1 to account for varying efficiency in different loci. Shown are technical replicates of n independent experiments. Grey and black bars represent the mean of the control and the respective small molecule, respectively. Concentrations used were 20 µM of NU7026, 0.01 µM of Trichostatin A (TSA), 0.05 µM of MLN4924, 1 µM of NSC 19630, 5 µM of NSC 15520, 20 µM of AICAR, 1 µM of RS-1, 1 µM of Resveratrol, 1 µM of SCR7, 5 µM of L755507, 5 µM of STL127685 and 20 µM of B02.

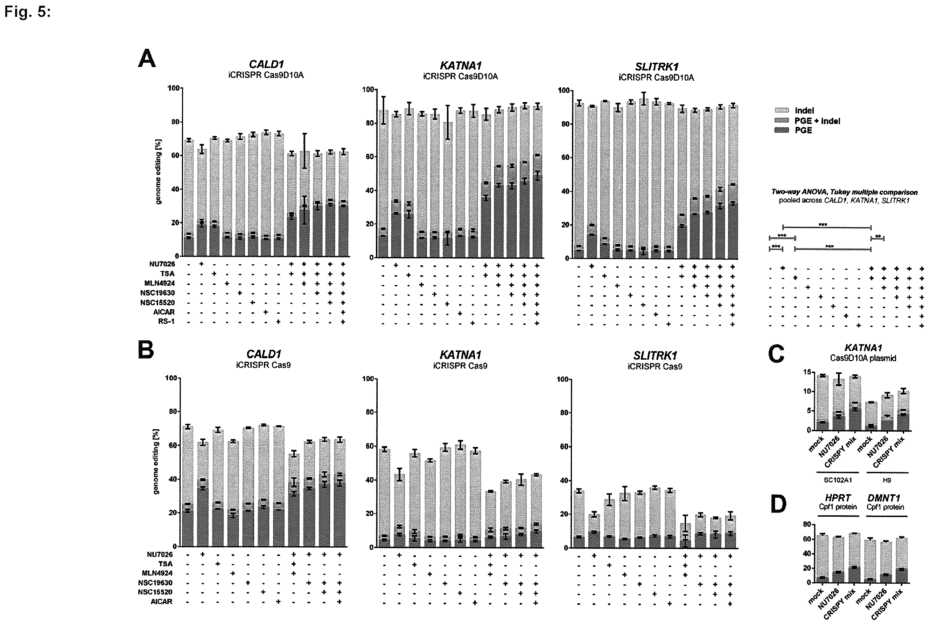

**Fig. 5****: Impact of small molecule combinations on precise genome editing (PGE) efficiency in *CALD1*, *KATNA1* and *SLITRK1* with Cas9D10A and Cas9, and in *HPRT* and *DMNT1* with Cpf1.** Small molecules have an additive effect on PGE efficiency with Cas9D10A (A) but not with Cas9 (B) in the 409-B2 iCRISPR iPSC lines. PGE efficiency of *KATNA1* was also increased in SC102A1 iPCs and H9 ESCs using the CRISPY mix with plasmid delivered Cas9D10A (C). PGE of *HPRT* and *DMNT1* in 409-B2 iPSCs with recombinant Cpf1 was increased using the CRISPY mix as well (D). Shown are PGE, PGE + indels, and indels with green, grey or blue bars, respectively. Error bars show the standard deviation of three technical replicates for A,B and D and two technical replicates for C. Concentrations used were 20 µM of NU7026, 0.01 µM of Trichostatin A (TSA), 0.05 µM of MLN4924, 1 µM of NSC 19630, 5 µM of NSC 15520, 20 µM of AlCAR and 1 µM of RS-1. CRISPY mix indicates a small molecule mix of NU7026, TSA, MLN4924 and NSC 15520. Significances of changes in Knock-In efficiencies were determined using a two-way-ANOVA and Tukey multiple comparison pooled across the three genes *CALD1*, *KATNA1, SLITRK1.* Genes and treatments were treated as random and fixed effect, respectively. Analysis included 3 technical replicates for each gene. P values are adjusted for multiple comparison (** P ≤ 0.01, *** P ≤ 0.001).

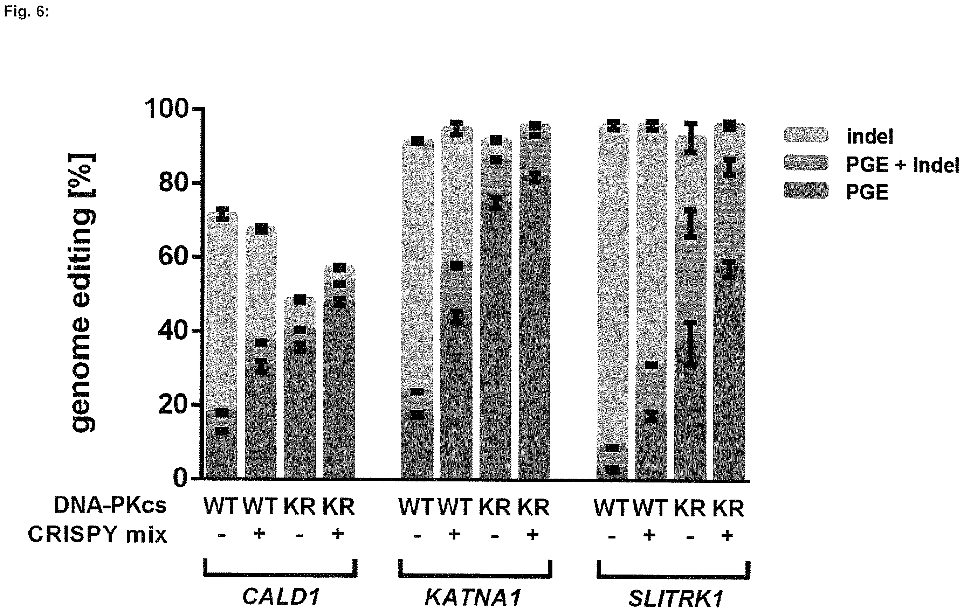

**Fig. 6****: Additive effect of CRISPY mix and DNA-PKcs (K3752R) on precise genome editing (PGE) efficiency in *CALD1*, *KATNA1* and *SLITRK1* with Cas9D10A**. The PGE efficiency is strongly increased with the DNA-PKcs KR mutant (KR) compared to the wildtype (WT) and further increased by addition of the CRISPY mix. Shown are PGE, PGE + indels, and indels with green, grey or blue bars, respectively. Error bars show the standard deviation of three technical replicates. CRISPY mix indicates a small molecule mix of 20 µM NU7026, 0.01 µM Trichostatin A (TSA), 0.05 µM MLN4924 and 5 µM NSC 15520.

### Methods

### Cell culture

iPSC lines cultured for this project included human 409-B2 iPSC (female, Riken BioResource Center) and SC102A1 iPSC (male, BioCat GmbH), as well as H9 ESC (female, WiCell Research Institute). Cells were grown on Matrigel Matrix (Corning, 35248) at 37°C in a humidified incubator gassed with 5% CO₂. mTeSR1 (StemCell Technologies, 05851) with mTeSR1 supplement (StemCell Technologies, 05852) was used as culture media. Media was replaced every day. Cell cultures were maintained 4-6 days until ∼ 80% confluency and subcultured at a 1:6 to 1:10 dilution. Cells were dissociated using EDTA (VWR, 437012C) for subculturing. The media was supplemented with 10 µM Rho-associated protein kinase (ROCK) inhibitor Y-27632 (Calbiochem, 688000) after cell splitting for one day in order to increase cell survival.

### Generation and validation of iCRISPR cell lines

Human 409-B2 iPSCs were used to create an iCRISPR-Cas9 line as described by Gonzalez et al. (Gonzalez et al. 2014). For the production of iCRISPR-Cas9D10A line Puro-Cas9 donor was subjected to site-directed mutagenesis with the Q5 mutagenesis kit (New England Biolabs, E0554S). Primers were ordered from IDT (Coralville, USA) and are shown in Table 2. Expression of the pluripotency markers SOX2, OCT-4, TRA1-60 and SSEA4 in iCRISPR lines was validated using the PSC 4-Marker immunocytochemistry kit (Molecular Probes, A24881) (data not shown). Quantitative PCR was used to confirm doxycycline inducible Cas9 or Cas9D10A expression and digital PCR was used to exclude off-target integration of the iCRISPR cassettes (data no shown).

### Small molecules

Commercially available small molecules used in this study were NU7026 (SIGMA, T8552), TSA (SIGMA, T8552), MLN4924 (Adooq BioScience, A11260), NSC 19630 (Calbiochem, 681647), NSC 15520 (ChemBridge, 6048069), AICAR (SIGMA, A9978), RS-1 (Calbiochem, 553510), Resveratrol (Selleckchem, S1396), SCR7 (XcessBio, M60082-2s), L755507 (TOCRIS, 2197), B02 (SIGMA, SML0364) and STL127685 (Vitas-M). STL127685 is a 4-fluorophenyl analog of the non-commercially available STL127705. Stocks of 15 mM (or 10 mM for NU7026) were made using dimethylsulfoxide (DMSO)(Thermo Scientific, D12345). Solubility is a limiting factor for NU7026 concentration. Suitable working solutions for different concentrations were made so that addition of each small molecule accounts for a final concentration of 0.08% (or 0.2% for NU7026) DMSO in the media. Addition of all small molecules would lead to a final concentration of 0.7% DMSO.

### Design of gRNAs and ssODNs

We chose to introduce one desired mutation in three genes *CALD1*, *KATNA1* and *SLITRK1* back to the state of the last common ancestor of human and Neanderthal (Prüfer et al. 2013). gRNA pairs for editing with the Cas9D10A nickase were selected to cut efficiently at a short distance from the desired mutation and from the respective partnering sgRNA. The efficiency was estimated with the sgRNA scorer 1.0 tool (Chari et al. 2015) as a percentile rank score. ssODN guides for nickase editing were designed to have the desired mutation and Cas9-blocking mutations to prevent re-cutting of the locus and had 50nt homology arms upstream and downstream of each nick (Fig. 2). gRNA of the nickase gRNA pair that cuts closer to the desired mutation was used for Cas9 nuclease editing together with a 90nt ssODN centered at the desired mutation and containing a Cas9-blocking mutation (Fig. 2). ssODNs for editing of *HPRT* and *DMNT1* using Cpf1 were designed to contain a blocking mutation near the PAM site and an additional mutation near the cut. gRNAs (crRNA and tracR) and ssODN were ordered from IDT (Coralville, USA). ssODNs and crRNA targets are shown in Table 2.

### Lipofection of oligonucleotides

Cells were incubated with media containing 2 µg/ml doxycycline (Clontech, 631311) 2 days prior to lipofection. Lipofection (reverse transfection) was done using the alt-CRISPR manufacturer's protocol (IDT) with a final concentration of 7.5 nM of each gRNA and 10 nM of the respective ssODN. In brief, 0.75 µl RNAiMAX (Invitrogen, 13778075) and the respective oligonucleotides were separately diluted in 25 µl OPTI-MEM (Gibco, 1985-062) each and incubated at room temperature for 5 min. Both dilutions were mixed to yield 50 µl of OPTIMEM including RNAiMAX, gRNAs and ssODNs. The lipofection mix was incubated for 20-30 min at room temperature. During incubation cells were dissociated using EDTA for 5 min and counted using the Countess Automated Cell Counter (Invitrogen). The lipofection mix, 100 µl containing 25.000 dissociated cells in mTeSR1 supplemented with Y-27632, 2 µg/ml doxycycline and the respective small molecule(s) to be tested were througoutly mixed and put in one well of a 96 well covered with Matrigel Matrix (Corning, 35248). Media was exchanged to regular mTeSR1 media after 24hours.

### Ribonucleoprotein nucleofection

The recombinant A.s. Cpf1 protein and electroporation enhancer was ordered from IDT (Coralville, USA) and nucleofection was done using the manufacturer's protocol, except for the following alterations. Nucleofection was done using the B-16 program of the Nucleofector 2b Device (Lonza) in cuvettes for 100 µl Human Stem Stell nucleofection buffer (Lonza, WPH-5022) containing 1 million 409-B2 iPSCs, 78 pmol electroporation enhancer, 0.3nmol gRNA, 200pmol ssODN donor, 252pmol Cpf1. Cells were counted using the Countess Automated Cell Counter (Invitrogen).

### FACS-sorting

Introduction of 2 µg plasmid DNA (pSpCas9n(BB)-2A-GFP (PX461), Addgene #48140) into cells not expressing Cas9 inducably was done using the B-16 program of the Nucleofector 2b Device (Lonza) in cuvettes for 100 µl Human Stem Stell nucleofection buffer (Lonza, WPH-5022) containing 1 million of either SC102A1 iPSC or H9 ESC. Cells were counted using the Countess Automated Cell Counter (Invitrogen). 24h after nucleofection cells were dissociated using Accutase (SIGMA, A6964), filtered to obtain a single cell solution, and subjected to fluorescence associated cell sorting (FACS) for GFP expressing cells. During sorting with the BD FACSAria III (Becton-Dickinson) cells were kept at 4°C in mTeSR1 supplemented with Y-27632. 48h after sorting cells were subjected to lipofection with sgRNAs, ssODNs and treatment with small molecules.

### Illuminalibrary preparation and sequencing

Three days after lipofection cells were dissociated using Accutase (SIGMA, A6964), pelleted, and resuspended in 15 µl QuickExtract (Epicentre, QE0905T). Incubation at 65°C for 10 min, 68°C for 5 min and finally 98°C for 5 min was performed to yield ssDNA as a PCR template. Primers for each targeted loci of *CALD1, KATNA1* and *SLITRK1* containing adapters for Illumina sequencing were ordered from IDT (Coralville, USA). PCR was done in a T100 Thermal Cycler (Bio-Rad) using the KAPA2G Robust PCR Kit (Peqlab, 07-KK5532-03) with supplied buffer B and 3 µl of cell extract in a total volume of 25 µl. The thermal cycling profile of the PCR was: 95°C 3 min; 34x (95° 15 sec, 65°C 15 sec, 72°C 15 sec); 72°C 60 sec. P5 and P7 Illuminaadapters with sample specific indices were added in a second PCR reaction (Kircher et al. 2012) using Phusion HF MasterMix (Thermo Scientific, F-531 L) and 0.3 µl of the first PCR product. The thermal cycling profile of the PCR was: 98°C 30 sec; 25x (98° 10 sec, 58°C 10 sec, 72°C 20 sec); 72°C 5 min. Amplifications were verified by size separating agarose gel electrophoresis using EX gels (Invitrogen, G4010-11). The indexed amplicons were purified using Solid Phase Reversible Immobilization (SPRI) beads (Meyer, Kircher 2010). Double-indexed libraries were sequenced on a MiSeq (Illumina) giving paired-end sequences of 2 x 150 bp. After base calling using Bustard (Illumina) adapters were trimmed using leeHom (Renaud et al. 2014).

### CRISPResso analysis

CRISPresso (Pinello et al. 2016) was used to analyse sequencing data from CRISPR genome editing experiments for percentage of wildtype, targeted nucleotide substitutions (TNS), indels and mix of TNS and indels. Parameters used for analysis were '-w 20', '-- min_identity_score 70' and '--ignore_substitutions' (analysis was restricted to amplicons with a minimum of 70% similarity to the wildtype sequence and to a window of 20bp from each gRNA; substitutions were ignored, as sequencing errors would be falsly characterized as NHEJ-events)

### Statistical analysis

Significances of changes in TNS efficiencies were determined using a two-way-ANOVA and Tukey multiple comparison pooled across the three genes *CALD1*, *KATNA1, SLITRK1.* Genes and treatments were treated as random and fixed effect, respectively. Hence, we tested the effect of treatment against its interaction with gene (Zar 1999). Analysis included 3 technical replicates for each gene. We checked for whether the assumptions of normally distributed and homogeneous residuals were fulfilled by visual inspection of a QQ-plot (Field 2005) and residuals plotted against fitted values (Quinn & Keough 2002). These indicated residuals to be roughly symmetrically distributed but with elongated tails (i.e., too large positive and negative residuals) and no obvious deviations from the homogeneity assumption. P values are adjusted for multiple comparison. Statistical analysis was done using R.

### Study design

We aimed to test the precise genome editing efficiency of several small molecules in iPSCs. The compounds SCR7, L755507 and RS-1 have been indicated in the literature as potential CRISPR-Cas effectors, whereas the other test compounds have not yet been known for this purpose. The test compounds are shown in Table 1.

**Table 1: Overview of the small molecules evaluated in this study.** The protein targeted, its function, as well as its respective pathway and the function of the small molecule are shown. Literature references with an asterisk indicate the small molecule as a CRISPR-Cas effector. Abbreviations: alternative NHEJ (Alt-NHEJ), damage dependent signaling (DDS).

| **Pathway** | **Protein** | **Protein function** | **Small molecule** | **Small molecule function** | **Reference** |
|---|---|---|---|---|---|
| **NHEJ** | **Ku70**/**80** | First proteins to bind to DNA ends | **STL127685** | 4-fluorophenyl analog of a Ku70/80 inhibitor | Weterings et al. 2016 |
| | **DNA-PK** | Complex of Ku70/80 and DNA-PKcs, DNA-PKcs phosphorylates itself and downstream effectors at the repair site | **NU7026** | DNA-PK inhibitor | Suzuki et al. 2016* |
| | **Ligase IV** | End-processing ligation | **SCR7** | Ligase IV inhibitor | Maruyama et al. 2015* |
| **Alt-NHEJ (NHEJ)** | **WRN helicase** | DNA unwinding | **NSC 19630** | WRN helicase inhibitor | Aggarwal et al. 2011 |
| **HDR** | **CtIP** | DNA end resection | **MLN4924** | NAE inhibitor, inhibts neddylation of CtIP | Jimeno et al. 2015 |
| | **RPA** | Coating and stabilization of ssDNA | **NSC15520** | Inhibits binding of RPA to p53 and RAD9 | Glanzer et al. 2011,2013 |
| | **RAD52** | ssDNA annealing | **AICAR** | RAD52 inhibitor | Sullivan et al. 2016 |
| | **RAD51** | Strand invasion with the donor DNA | **RS-1** | RAD51 enhancer | Song et al. 2016* |
| | | | **B02** | RAD51 inhibitor | Huang et al. 2011 |
| **DDS** | **ATM** | Phosphorylates many DNA repair proteins | **Resveratrol** | Direct stimulatory effects on purified ATM | Lee et al. 2014 |
| | | | **TSA** | Histone deacetlyase inhibitor, induces phosphorylation of Ser1981 in ATM | Lee 2007 |
| **?** | **β3-adrenergic receptor** | Involved in activation of adenylate cyclase | **L755507** | β3-adrenergic receptor agonist | Yu et al. 2015' |

As a tool for analysis of effects of small molecules on HDR efficiency, we generated an iCRISPR-Cas9 and iCRISPR-Cas9D10A iPSC line that allows for doxycycline inducible Cas9 or Cas9D10A expression and efficient delivery of ssODN and guide RNAs (gRNAs) (Gonzalez et al. 2014). The genome editing and analysis flowchart is shown in Figure 1. The design of gRNAs, ssODNs and Primers used in the editing of three example genes *CALD1*, *KATNA1* and *SLITRK1* is shown in Figure 2 and Table 2.

**Table 2: Oligonucleotides used in this study.** gRNA (crRNA target) and single stranded DNA donors (ssODNs) for editing of *CALD1, KATNA1, SLITRK1, HPRT* and *DMNT1* as well as primers for analysis and Q5 site-directed-mutagenesis of the Cas9 iCRISPR donor plasmid are shown. Mutations are in bold letters and ancestral mutations are underlined as well.

| | | |
|---|---|---|
| **gRNAs** | *CALD1* t1 | TGGAGACTATTGCTGCTTGA (SEQ ID NO.1) |
| | *CALD1* t2 | GCAGTATACCAGTGCAATTG (SEQ ID NO.2) |
| | *KATNA1* t1 | AAATGATGACCCTTCCAAAA (SEQ ID NO.3) |
| | *KATNA1* t2 | CAACACCTAAAATAAGGGTA (SEQ ID NO.4) |
| | *SLITRK1* t1 | GCTAACAGTTTACCCTGCCC (SEQ ID NO.5) |
| | *SLITRK1* t1 | ACCCGTCGCTATCGCTGCTG (SEQ ID NO.6) |
| | *HPRT* t1 | GGTTAAAGATGGTTAAATGAT (SEQ ID NO.7) |
| | *DMNT1* t1 | CTGATGGTCCATGTCTGTTAC (SEQ ID NO.8) |
| **ssODNs** | *CALD1* Cas9 | GTATACTGCTCCAGTCTGCTGTCAATCTTGGAGACTACTGCTGCTTGATGGGTCGATTTGA CACCACTGCTAAAAAAGTAAACACATACA (SEQ ID NO.9) |
| | *CALDI* Cas9D10A | TTATATGTATGTGTTTACTTTTTAGCAGTGGTGTCAAATCGACCCATCAAGCTGCAGTAG TCTCCAAGATTGACAGCAGACTGGAGCAGTATACCAGTGCTATTGAGGTGAGAATTGTCCT CAGCGTTATGGTCCTGCTGAACAGAAATAGA (SEQ ID NO.10) |
| | *KATNA1* Cas9 | GAAGGGTCATCATTTTCAGAAGCACCTCCAACACCTAAAATAAGGGAAAGGGGAGAGTGAA AAAGATATTAAGTTGGATTATACCAAATG (SEQ ID NO.11) |
| | *KATNA1* Cas9D10A | CTCATCTATATCCCAGGGAAAATTAGTAGCTGCCAGAACCATAACCATTTTAGAAGGGTCA TCATTTTCAGAGGCGCCTCCAACACCTAAAATAACGGTAAGGGGAGAGTGAAAAAGATATT AAGTTGGATTATACCAAATGAAGCT (SEQ ID NO.12) |
| | *SLITRK1* Cas9 | TGTTAGCTAAGGGTTTGTTCCTGGCGCTACCCGTCGCTATCGCTGCGGTGGGTCTGATTTT GATCTGCCAGTTGCCTGGGATCTTTGTAC (SEQ ID NO.13) |
| | *SLITRK1* Cas9D10A | TCATCTTTAAACCCGACCCTGGGATGTGGTCGCAGCTGCAGCCCCCAGGACAGGGTAAACT GTTAGCTAAGGGTTTGTTCCTGGCACTGCCCGTCGCTATCGCTGCGGTGGTCTGATTTTG ATCTGCCAGTTGCCTGGGATCTTTGTACCTCCG (SEQ ID NO.14) |
| | *HPRT* Cpf1 | GCCATTTCACATAAAACTCTTTTAGGTTATAGATGGTTAAATGAATGACAAAAAAAGTAAT TCACTTACAGTCTGGCTTATATCCAACAC (SEQ ID NO.15) |
| | *DMNT1* Cpf1 | TTAACATCAGTACGTTAATGTTTCCTGATCGTCCATGTCTGTTAGTCGCCTGTCAAGTGGC GTGACACCGGGCGTGTTCCCCAGAGTGAC (SEQ ID NO.16) |
| **Primers** | *CAD1* forward | ACACTCTTTCCCTACACGACGCTCTTCCGATCTGCTAATCAGCTAGCATATGTATGAGAA (SEQ ID NO.17) |
| | *CALD1* reverse | GTGACTGGAGTTCAGACGTGTGCTCTTCCGATCTTTGGACTTGATTATTGTCCTAAGTG (SEQ ID NO.18) |
| | *KATNA1* forward | ACACTCTTTCCCTACACGACGCTCTTCCGATCTCCTGACGGCAAAGGAATATAG (SEQ ID NO.19) |
| | *KATNA1* reverse | GTGACTGGAGTTCAGACGTGTGCTCTTCCGATCTACTGTGCTTCCTTGTATTGTTGT (SEQ ID NO.20) |
| | *SLITRK1* forward | ACACTCTTTCCCTACACGACGCTCTTCCGATCTGGGCTTCAAATCAGCCAAG (SEQ ID NO.21) |
| | *SLITRK1* reverse | GTGACTGGAGTTCAGACGTGTGCTCTTCCGATCTTTTCAAGACAAATGGGCAAG (SEQ ID NO.22) |
| | *HPRT forward* | ACACTCTTTCCCTACACGACGCTCTTCCGATCTGGTGAAAAGGACCCCACGAA (SEQ ID NO.23) |
| | *HPRT* reverse | GTGACTGGAGTTCAGACGTGTGCTCTTCCGATCTTGGCAAATGTGCCTCTCTACAAAT (SEQ ID NO.24) |
| | *DMNT1* forward | ACACTCTTTCCCTACACGACGCTCTTCCGATCTTGAACGTTCCCTTAGCACTCTG (SEQ ID NO.25) |
| | *DMNT1* reverse | GTGACTGGAGTTCAGACGTGTGCTCTTCCGATCTCCTTAGCAGCTTCCTCCTCC (SEQ ID NO.26) |
| | Q5 D10A forward | TGGTGCCGAT**AG**CCAGGCCGATG (SEQ ID NO.27) |
| | Q5 D10A reverse | ACTCTGTGGGCTGGGCCG (SEQ ID NO.28) |

Small molecules that we chose to test for their ability to block NHEJ are SCR7, STL127685, which is a 4-fluorophenyl analog of the Ku70/80 inhibitor STL127705 (Weterings et al. 2016), and DNA-PK inhibitor NU7026 (Suzuki et al. 2016). To block Alt-NHEJ we chose WRN helicase inhibitor NSC 19630 (Aggarwal et al. 2011). The NEDD8 activating enzyme (NAE) inhibitor MLN4924 has been shown to inhibit the neddylation of CtIP, which is increasing the extend of DNA end resection at strand breaks, and therefore regulates DNA double strand break repair pathway choice to preferentially undergo HDR (Jimeno et al. 2015). DNA resection leaves ssDNA which is coated and stabilized by RPA, before undergoing homology search and recombination. We assumed increasing availability of RPA could favor HDR. Fumaropimaric acid (NSC15520) prevents the association of RPA to p53 and RAD9 (Glanzer et al. 2011)(Glanzer et al. 2013), possibly increasing the abundance of available RPA for ssDNA. RAD51 is critical for homologous recombination with double stranded DNA (dsDNA), while RAD52 is needed for annealing of ssDNA (Grimme at al. 2010). We therefore chose to test the effect of RAD52 inhibitor AICAR (Sullivan et al. 2016), RAD51 inhibitor B02 (Huang et al. 2011), and RAD51 enhancer RS-1 (Song et al. 2016) on precise genome editing efficiency. Furthermore, we included small molecules that have been described to enhance the repair protein kinase ATM. Resveratrol has been described to have a direct stimulatory effect on the catalytic efficiency of purified ATM *in vitro* (Lee et al. 2014). The histone deacetylase inhibitor Trichostatin A actives an ATM-dependent DNA damage signaling pathway and has been described to increase HR (Lee 2007)(Jimeno et al. 2015). Lastly, we included ß3-adrenergic receptor agonist L755507 in the screen.

### Results

First we tested different concentrations of the small molecules to examine their influence on precise editing of *CALD1*, *KATNA1* and *SLITRK1* with iCRISPR Cas9D10A (Fig. 3). Consequently, we chose the respective concentration that gave the highest frequency of precise genome editing. When different concentrations had a similar effect on editing we chose the lower concentration. Effects of chosen small molecule concentrations on precise genome editing efficiency in *CALD1, KATNA1* and *SLITRK1* with Cas9D10A and Cas9 from repeated independent experiments are shown in Figure 4.

NU7026 treatment increased PGE for all three loci with Cas9D10A (Fig. 4A) and Cas9 (Fig. 4B). The mean change was 1.5-fold for *CALD1,* 2.6-fold for *KATNA1* and 2.5-fold for *SLITRK1* with Cas9D10A, and 1.5-fold for *CALD1,* 1.6-fold for *KATNA1* and 1.2-fold for *SLITRK1* with Cas9. In addition to NU7026, we found TSA and MLN4924 to have an enhancing effect on PGE frequency with Cas9D10A. TSA increased PGE 1.5-fold for *CALD1,* 2.2-fold for *KATNA1* and 1.8-fold for *SLITRK1* with Cas9D10A; interestingly no enhancing effect was found with Cas9. MLN4924 increased PGE 1.2-fold for *CALD1*, 1.1-fold for *KATNA1* and 1.3-fold for *SLITRK1* with Cas9D10A. With Cas9 there is a slight diminishing effect of MLN4924 on PGE. Treatment with NSC 15520 increased PGE of *CALD1* with Cas9D10A and Cas9 1.4 fold and 1.3 fold, respectively. However, there was no effect on PGE of *KATNA1* and *SLITRK1.* NSC 19630, AICAR, RS-1, Resveratrol, SCR7, L755507 and STL127685 showed no clear effect on PGE frequency over the three genes *CALD1*, *KATNA1* and *SLITRK1* compared to the respective controls with Cas9D10A and no effect or a diminishing effect with Cas9. B02 halved PGE in all three loci both with Cas9D10A and Cas9.

We next tested if combinations of small molecules would have an enhancing effect on PGE with Cas9D10A or Cas9. We chose small molecules that showed an increase in PGE with Cas9D10A in at least one gene and never a PGE decrease; those molecules are: NU7026, TSA, MLN4924, NSC 19630, NSC 15520, AICAR and RS-1.

Impact of small molecule combinations on editing of the tree different loci with either Cas9D10A or Cas9 are shown in Fig. 5. For editing with Cas9D10A treatment with NU7026 or PGE resulted in 2.3- or 1.8-fold higher PGE frequency than without the molecules (Tukey's pair-wise post-hoc comparisons: p<0.001) (Fig. 5A). A combination of both NU7026 and TSA resulted in 1.3 or 1.6 times higher PGE frequency than either NU7026 or TSA alone (p<0.001). Addition of MLN4924 to the mix of NU7026 and TSA lead to an additional 1.3-fold increase in PGE (p<0.01). Further addition of NSC 15520 slightly increased the mean of PGE for all loci when using Cas9D10A for editing, without reaching statistical significance. Addition of NSC 19630, AICAR and RS-1 had no measurable effect on PGE.

We conclude that the mix of small molecules that increases the frequency of PGE with Cas9D10A the most is a combination of NU7026 (20µM), TSA (0.01µM), MLN4924 (0.5µM) and NSC 15520 (5µM), which we name 'CRISPY' nickase mix. It yielded an increase of PGE of 2.8-fold (from 11 to 31%) for *CALD1*, 3.6-fold (from 12.8 to 45.8%) for *KATNA1* and 6.7-fold (from 4.7 to 31.6%) for *SLITRK1* in the iCRISPR 409-B2 iPSC line.

To test if the CRISPY mix can be used in other human pluripotent stem cell lines we edited one gene *KATNA1* in SC102A1 iPSCs and H9 ESCs using Cas9D10A plasmid nucleofection and found that PGE was increased 2.6-fold and 2.8-fold, respectively (Fig. 5C).

When using Cas9 we found no significant beneficial effect of adding other small molecules on top of NU7026 (Fig. 5B). Therefore we tested the impact of the CRISPY mix on PGE efficiency when using Cpf1, which introduces staggered cuts like the Cas9D10A double nicking approach. Using the CRISPY mix together with Cpf1 ribonucleoprotein nucleofection in 409-B2 hiPSCs the PGE efficiency increased 2.9 fold for *HPRT* and 4 fold for *DMNT1* compared to the control (Fig. 5D). Addition of only NU7026 increased PGE 2.1 fold for *HPRT* and 2.4 fold for *DMNT1.*

Chemical inhibiton of DNA-PK did increase PGE efficiency with Cas9D10A, Cas9 and Cpf1. Next, we mutated DNA-PKcs (K3753R) to be catalytically inactive in our iCRISPR nickase cell line. It has been shown that inactivation of a kinase active site of DNA-PKcs (K3753R) increases HDR six to ten fold in CHO V3 cells (Shrivastav et al. 2009). HDR is directly or indirectly enhanced in cells expressing DNA-PKcs-KR by activation of ATM kinase and not only due to passive shunting from NHEJ to HDR (Shrivastav et al. 2009). Arguably, because NHEJ will be repressed, cells that have a DSB and cannot be repaired by HDR will die and repair might be further enhanced by the CRISPY mix with Cas9D10A. Indeed, the DNA-PKcs mutant had a 2.8-fold, 4,3-fold and 12.4-fold increase of PGE for *CALD1*, *KATNA1* and *SLITRK1,* respectively (see Figure 6). In addition with the CRISPY mix the efficiency of PGE compared to the DNA-PKcs wildtype without small molecule treatment was increased even further. The PGE efficiency was 48% (3.7-fold increase), 82% (4.7-fold increase) and 57.5% (19.2-fold increase) for *CALD1*, *KATNA1* and *SLITRK1,* respectively.

### Discussion

Small molecules that increased PGE in iPSCs with Cas9D10A and ssODN donor both alone (Fig. 4A) and in a mix (Fig. 5A) are NU7026, TSA, MLN4924 and NSC 15520. The mix also shows an additional increasing effect on PGE with Cpf1 but not with Cas9 compared to single treatment with NU7026. NU7026 inhibits DNA-PK, a major complex in NHEJ pathway (Shrivastav et al. 2008) and has been previously shown to increase PGE efficiency in hiPSCs (Suzuki et al. 2016). TSA can activate an ATM-dependent DNA damage signaling pathway (Lee 2007). The Nedd8 activating enzyme (NAE) inhibitor MLN4924 has been shown to inhibit the neddylation of CtIP which is increasing the extent of DNA end resection at strand breaks thereby promoting HDR (Jimeno et al. 2015). DNA resection leaves ssDNA which is coated and stabilized by RPA before undergoing homology search and recombination. NSC15520 prevents the association of RPA to p53 and RAD9 (Glanzer et al. 2011)(Glanzer et al. 2013), possibly increasing the abundance of available RPA for ssDNA which could favor HDR. RS-1, SCR7 and L755507 for which there are conflicting reports on their capacity to increase PGE showed no clear effect in our hands on PGE neither with Cas9 nor with Cas9D10A.

The enhancing effect of TSA and MLN4924 on PGE when used with Cas9D10A double nicking or Cpf1 contrary to no effect visible with Cas9 suggests that blunt and staggered DNA cuts (5' overhangs) are repaired by different repair mechanisms. To date the mechanism of HDR with ssODN is not clear but models have been proposed (Bothmer et al. 2017). Bothmer et al. find that 5' overhang structures yield higher levels of HDR than 3' overhangs and suggest that different overhang polarities engage different repair pathways.

However, the negative effect of MLN4924 with Cas9 editing might be due to shorter ssODN used compared to longer Cas9D10A ssODN. If extensive DNA resection triggered by MLN4924 takes place, there are is no homology left for ssODN annealing if short donors are used. Since long 5'overhangs are present in Cas9D10A editing, homologous DNA is provided, even after extensive resection. Therefore, usage of longer donors might render MLN4924 effective in enhancing precise genome editing also when using Cas9.

While RAD51 is obviously important for classical homologous recombination with dsDNA (Shrivastav et al. 2008) we wanted to examine if RAD52, rather than RAD51, could be the driving force behind HDR of ssODN since RAD52 is needed for annealing of ssDNA (Grimme et al. 2010). Our results of the effect of RAD52 inhibitor AICAR, RAD51 inhibitor B02, and RAD51 enhancer RS-1 on PGE efficiency suggests that RAD51 and not RAD52 is important for precise editing with ssODN, since inhibition of RAD51 by B02 halved and inhibition of RAD52 had no effect on PGE efficiency. Interestingly, stimulation of RAD51 with RS-1 had no beneficial effect on PGE.

To increase PGE efficiency we produced an inducible Cas9D10A iPSC line and optimized the delivery of ssODNs such that sometimes indels in more than 90% of the cells are observed (Fig. 3A). Using the CRISPY small molecule mix we achieve almost 50% PGE in hiPSCs (Fig. 3A), the highest PGE efficiency of human pluripotent stem cells described to date to our knowledge. Furthermore, we are the first to show efficient PGE by using Cpf1 (20%) with help of the CRISPY mix (Fig. 5D). The CRISPY mix provides an easy tool for increasing PGE frequency and could therefore be useful for many researchers or medical professionals using the CRISPR system to perform precise genome editing. Together with catalytically inactive DNA-PKcs, this finding has the potential to revolutionize CRISPR assisted research and tailored genomes, since editing of multiple loci at once is possible. Not only is the editing efficiency very high (up to 82%) - the limited proportion of indels now allows for several rounds of editing, thereby increasing the chance of donor Knock-In. Researchers can benefit from this finding either by mutating DNA-PKcs in the cell line to be edited, or by exogenously administered DNA-PKcs K3753 (e.g. plasmid, recombinant protein) that will overwhelm the endogenous DNA-PKcs (e.g. in combination with siRNAs).

### References

1. Aggarwal, M., Sommers, J.A., Shoemaker, R.H. & Brosh, R.M., Jr. Inhibition of helicase activity by a small molecule impairs Werner syndrome helicase (WRN) function in the cellular response to DNA damage or replication stress. Proc Natl Acad Sci U S A 108, 1525-1530 (2011).
2. Chari, R., Mali, P., Moosburner, M. & Church, G.M. Unraveling CRISPR-Cas9 genome engineering parameters via a library-on-library approach. Nat Methods 12, 823-826 (2015).
3. Chu, V.T. et al. Increasing the efficiency of homology-directed repair for CRISPR-Cas9-induced precise gene editing in mammalian cells. Nat Biotechnol 33, 543-548 (2015).
4. Field, A. Discovering Statistics using SPSS. (Sage Publications, London; 2005).
5. Fu, Y. et al. High-frequency off-target mutagenesis induced by CRISPR-Cas nucleases in human cells. Nat Biotechnol 31, 822-826 (2013).
6. G., D.R.I. Alternative pathways of non-homologous end joining (NHEJ) in genomic instability and cancer. Transl Cancer Res 2 (2013).
7. Glanzer, J.G. et al. A small molecule directly inhibits the p53 transactivation domain from binding to replication protein A. Nucleic Acids Res 41, 2047-2059 (2013).
8. Glanzer, J.G., Liu, S. & Oakley, G.G. Small molecule inhibitor of the RPA70 N-terminal protein interaction domain discovered using in silico and in vitro methods. Bioorg Med Chem 19, 2589-2595 (2011).
9. Gonzalez, F. et al. An iCRISPR platform for rapid, multiplexable, and inducible genome editing in human pluripotent stem cells. Cell Stem Cell 15, 215-226 (2014).
10. Greco, G.E. et al. SCR7 is neither a selective nor a potent inhibitor of human DNA ligase IV. DNA Repair (Amst) 43, 18-23 (2016).
11. Grimme, J.M. et al. Human Rad52 binds and wraps single-stranded DNA and mediates annealing via two hRad52-ssDNA complexes. Nucleic Acids Res 38, 2917-2930 (2010).
12. Huang, F. et al. Identification of specific inhibitors of human RAD51 recombinase using high-throughput screening. ACS Chem Biol 6, 628-635 (2011).
13. Jimeno, S. et al. Neddylation inhibits CtIP-mediated resection and regulates DNA double strand break repair pathway choice. Nucleic Acids Res 43, 987-999 (2015).
14. Kim, D. et al. Genome-wide analysis reveals specificities of Cpf1 endonucleases in human cells. Nat Biotechnol 34, 863-868 (2016).
15. Kircher, M., Sawyer, S. & Meyer, M. Double indexing overcomes inaccuracies in multiplex sequencing on the Illumina platform. Nucleic Acids Res 40, e3 (2012).
16. Kleinstiver, B.P. et al. Genome-wide specificities of CRISPR-Cas Cpf1 nucleases in human cells. Nat Biotechnol 34, 869-874 (2016).
17. Lee, J.H., Guo, Z., Myler, L.R., Zheng, S. & Paull, T.T. Direct activation of ATM by resveratrol under oxidizing conditions. PLoS One 9, e97969 (2014).
18. Lee, J.S. Activation of ATM-dependent DNA damage signal pathway by a histone deacetylase inhibitor, trichostatin A. Cancer Res Treat 39, 125-130 (2007).
19. Lin, S., Staahl, B.T., Alla, R.K. & Doudna, J.A. Enhanced homology-directed human genome engineering by controlled timing of CRISPR/Cas9 delivery. Elife 3, e04766 (2014).
20. Maruyama, T. et al. Increasing the efficiency of precise genome editing with CRISPR-Cas9 by inhibition of nonhomologous end joining. Nat Biotechnol 33, 538-542 (2015).
21. Meyer, M. & Kircher, M. Illumina sequencing library preparation for highly multiplexed target capture and sequencing. Cold Spring Harb Protoc 2010, pdb prot5448 (2010).
22. Milanowska, K. et al. REPAIRtoire--a database of DNA repair pathways. Nucleic Acids Res 39, D788-792 (2011).
23. Nussenzweig, A. & Nussenzweig, M.C. A backup DNA repair pathway moves to the forefront. Cell 131, 223-225 (2007).
24. Pinder, J., Salsman, J. & Dellaire, G. Nuclear domain 'knock-in' screen for the evaluation and identification of small molecule enhancers of CRISPR-based genome editing. Nucleic Acids Res 43, 9379-9392 (2015).
25. Pinello, L. et al. Analyzing CRISPR genome-editing experiments with CRISPResso. Nat Biotechnol 34, 695-697 (2016).
26. Prufer, K. et al. The complete genome sequence of a Neanderthal from the Altai Mountains. Nature 505, 43-49 (2014).
27. Quinn, G.P.K., M.J. Experimental Designs and Data Analysis for Biologists. (Cambridge University Press, Cambridge; 2002).
28. Renaud, G., Stenzel, U. & Kelso, J. leeHom: adaptor trimming and merging for Illumina sequencing reads. Nucleic Acids Res 42, e141 (2014).
29. Robert, F., Barbeau, M., Ethier, S., Dostie, J. & Pelletier, J. Pharmacological inhibition of DNA-PK stimulates Cas9-mediated genome editing. Genome Med 7, 93 (2015).
30. Shen, B. et al. Efficient genome modification by CRISPR-Cas9 nickase with minimal off-target effects. Nat Methods 11, 399-402 (2014).
31. Shrivastav, M., De Haro, L.P. & Nickoloff, J.A. Regulation of DNA double-strand break repair pathway choice. Cell Res 18, 134-147 (2008).
32. Shrivastav, M. et al. DNA-PKcs and ATM co-regulate DNA double-strand break repair. DNA Repair (Amst) 8, 920-929 (2009).
33. Song, J. et al. RS-1 enhances CRISPR/Cas9- and TALEN-mediated knock-in efficiency. Nat Commun 7, 10548 (2016).
34. Sullivan, K. et al. Identification of a Small Molecule Inhibitor of RAD52 by Structure-Based Selection. PLoS One 11, e0147230 (2016).
35. Suzuki, K. et al. In vivo genome editing via CRISPR/Cas9 mediated homology-independent targeted integration. Nature 540, 144-149 (2016).
36. Wang, K. et al. Efficient Generation of Orthologous Point Mutations in Pigs via CRISPR-assisted ssODN-mediated Homology-directed Repair. Mol Ther Nucleic Acids 5, e396 (2016).
37. Weterings, E. et al. A novel small molecule inhibitor of the DNA repair protein Ku70/80. DNA Repair (Amst) 43, 98-106 (2016).
38. Yang, D. et al. Enrichment of G2/M cell cycle phase in human pluripotent stem cells enhances HDR-mediated gene repair with customizable endonucleases. Sci Rep 6, 21264 (2016).
39. Yu, C. et al. Small molecules enhance CRISPR genome editing in pluripotent stem cells. Cell Stem Cell 16, 142-147 (2015).
40. Zar, J.H. Biostatistical Analysis. (Prentice Hall, New Jersey; 1999).
41. Zetsche, B. et al. Cpf1 is a single RNA-guided endonuclease of a class 2 CRISPR-Cas system. Cell 163, 759-771 (2015).
42. Zhang, J.P. et al. Efficient precise knockin with a double cut HDR donor after CRISPR/Cas9-mediated double-stranded DNA cleavage. Genome Biol 18, 35 (2017).

## Claims

1. A combination comprising at least 2 of:
(a) a compound (I) which is an HDAC inhibitor,
(b) a compound (II) which is an NAE inhibitor,
(c) a compound (III) which is a DNA-PK inhibitor, and
(d) a compound (IV) which is an RPA inhibitor.

2. The combination of claim 1,
wherein the compound (I) is Trichostatin A, and/or
the compound (II) is MLN4924, and/or
the compound (III) is NU7026 and/or
the compound (IV) is NSC15520.

3. The combination of claim 1 or 2, comprising
(i) at least one compound (I) and at least one compound (II),
(ii) at least one compound (I) and at least one compound (III),
(iii) at least one compound (I) and at least one compound (IV),
(iv) at least one compound (II) and at least one compound (III),
(v) at least one compound (II) and at least one compound (IV), or
(vi) at least one compound (III) and at least one compound (IV).

4. The combination of any one of claims 1-3, comprising
(i) at least one compound (I), at least one compound (II), and at least one compound (III),
(ii) at least one compound (I), at least one compound (II), and at least one compound (IV), or
(iii) at least one compound (II), at least one compound (III), and at least one compound (IV).

5. The combination of any one of claims 1-4, comprising at least one compound (I), at least one compound (II), at least one compound (III), and at least one compound (IV).

6. The combination of any one of claims 1-5 further comprising
(i) a DNA protein kinase catalytic subunit which is catalytically inactive, but structurally intact, particularly the mutated subunit K3753R,
(ii) a nucleic acid molecule encoding the DNA protein kinase catalytic subunit of (i) and/or
(iii) a eukaryotic cell capable of expressing the DNA protein kinase catalytic subunit of (i).

7. The combination of any one of claims 1-6 for use in genome editing in a eukaryotic target cell, particularly in a mammalian target cell, more particularly in a human target cell.

8. The combination of any one of claims 1-7 for use in genome editing in a stem cell, including an induced or embryonic pluripotent stem cell of an eukaryotic target organism, particularly a human induced or embryonic pluripotent stem cell.

9. The combination of any one of claims 1-6 for use according to claim 7 or 8, wherein the genome editing comprises introducing a staggered cut, particularly a staggered cut with 5' overhangs into the double-stranded genome of the target cell.

10. The combination of any one of claims 1-6 for use according to claim 9 wherein the genome editing comprises (i) the presence of a CRISPR/Cas9D10A enzyme in the target cell, or (ii) the presence of a CRISPR/Cpf1 enzyme in the target cell.

11. The combination of any one of claims 1-6 for use according to any one of claims 7-10, wherein the genome editing comprises introducing a donor DNA molecule carrying a desired mutation, which is a single-stranded or double-stranded DNA molecule, particularly a single-stranded DNA molecule, into the target cell.

12. The combination of any one of claims 1-5 for use in medicine including human or veterinary medicine.

13. Use of
(i) a DNA protein kinase catalytic subunit which is catalytically inactive, but structurally intact, particularly the mutated subunit K3753R,
(ii) a nucleic acid molecule encoding the DNA protein kinase catalytic subunit of (i) and/or
(iii) a eukaryotic cell capable of expressing the DNA protein kinase catalytic subunit of (i)
for genome editing in a eukaryotic target cell, particularly in a mammalian target cell, e.g. a human target cell including a human stem cell.

14. The use of claim 13 further comprising introducing a compound (I), a compound (II), a compound (III), a compound (IV), or a compound (V) as defined in claim 1 or a combination according to any one of claims 1-5 into the target cell.

15. A method for editing the genome of a eukaryotic target cell or target organism comprising introducing a combination according to any one of claims 1-5 into the target cell.
